(19) Europäisches Patentamt
European Patent Office
Office européen des brevets

(11) Publication number: 0 251 679 A2

# (12) EUROPEAN PATENT APPLICATION

(21) Application number: 87305621.2

(51) Int. Cl.$^4$: A61K 7/48

(22) Date of filing: 24.06.87

(30) Priority: 30.06.86 US 880466

(43) Date of publication of application:
07.01.88 Bulletin 88/01

(84) Designated Contracting States:
DE FR GB

(71) Applicant: DOW CORNING CORPORATION

Midland Michigan 48640(US)

(72) Inventor: Keil, Joseph Woodward
5805 Flaxmoor
Midland§Michigan(US)
Inventor: Rentsch, Stefan Felix
5800 Siebert
Midland Michigan(US)

(74) Representative: Lewin, John Harvey et al
Elkington and Fife High Holborn House 52/54
High Holborn
London WC1V 6SH(GB)

## (54) Stick vehicle for skin care substances.

(57) The invention is a stable mixture of alcohols and volatile silicone which is gelled by sodium stearate to provide a solid, shaped vehicle for topical delivery of cosmetics and other skin care substances to skin. The stick vehicle typically contains water, monohydric alcohol, a polyhydric alcohol, cyclic polydimethylsiloxanes, a polydiorganosiloxane-polyoxyalkylene copolymer, and sodium stearate.

EP 0 251 679 A2

# STICK VEHICLE FOR SKIN CARE SUBSTANCES

This invention relates to solid, shaped vehicles, commonly referred to as sticks, which are used for the topical application to skin of personal care substances such as deodorants or fragrances. More specifically, the invention relates to stable alcohol-silicone mixtures which are gelled by sodium salts of higher fatty acids such as sodium stearate.

Stick vehicles are well known in the cosmetic art for the application of active components such as antiperspirants, bacteriostats, moisturizers, and fragrances to skin. Sticks offer advantages relative to ease of application and convenience in comparison to liquids, creams, or sprays.

Generally, vehicles used for cosmetics are solidified to form sticks in either of two different ways. Simple and inexpensive sticks such as deodorant or perfume sticks are formed by gelling alcoholic solutions, typically ethanol and propylene glycol, with sodium salts of higher fatty acids such as sodium stearate. Antiperspirant sticks, however, are usually solidified by incorporating a mixture of solid and liquid waxes such as fatty acids, fatty esters, and fatty alcohols into the formulation. Sodium stearate is not used to solidify antiperspirant formulations because it reacts with the commonly used antiperspirant salt, aluminium chlorhydrate, to form an insoluble aluminium soap precipitate.

Early stick vehicles were often objectionably sticky or tacky and produced an unpleasant feel when applied to the skin. Newer formulations have been developed to avoid such undesirable characteristics. In particular, the acceptance of antiperspirant sticks by consumers has increased substantially because of the additional improvements in aesthetics and reduction in tackiness achieved by incorporating volatile cyclic polydimethylsiloxanes in the stick formulations. For example, Keil, in U.S. Patent No. 4,265,878, describes antiperspirant stick compositions comprising an aqueous solution of antiperspirant salts dispersed in a solid matrix of volatile cyclic polydimethylsiloxanes, a solid alkanoic acid, a waxy ester or a mixture of a waxy ester and a solid fatty alcohol, and polydiorganosiloxane-polyoxyalkylene copolymer.

There is also a need to improve the aesthetics by reducing the tackiness of the simpler sodium stearate gelled sticks. However, volatile cyclic polydimethylsiloxanes alone are not soluble in the alcoholic solutions which may be gelled by sodium stearate. To overcome this problem, DiSapio et al., in a commonly assigned U.S. Patent Application Serial No. 761,270, filing date August 1, 1985, describe the use of liquid fatty alcohols and polyoxyalkylated fatty alcohols as cosolvent components to compatibilize volatile silicone in other materials. For example, a transparent stick is described containing 14.9 parts of propylene glycol, 7.5 parts ethanol, 10 parts of PPG-10 cetyl ether, 19.8 parts of isostearyl alcohol, 39.8 parts of decamethylcyclopentasiloxane, and 8 parts of sodium stearate.

While the compositions of DiSapio et al. show much improved aesthetics, there is still a need to develop more efficient ways of incorporating volatile silicones into sodium stearate gelled stick vehicles for skin care substances.

It is an object of the invention to provide new stick vehicles that are useful as topical delivery systems for skin care substances, are gelled by sodium salts of higher fatty acids such as sodium stearate, and contain sufficient volatile silicone to improve aesthetics and reduce tackiness.

The present invention relates to a stick vehicle comprising from 15 to 70 percent by weight of an aqueous-alcohol mixture, from 10 to 70 percent by weight of cyclic polydimethylsiloxane, from 3 to 10 percent by weight of sodium stearate, and from 0.5 to 10 percent by weight of polydiorganosiloxane-polyoxyalkylene copolymer, with the added requirement that the amount of copolymer in the vehicle is at least 5 percent of the weight of cyclic polydimethylsiloxane. The aqueous-alcohol mixture consists essentially of 20 to 70 percent by weight of a polyhydric alcohol having 2 or 3 carbon atoms, 0 to 68.5 percent by weight of a monohydric alcohol with 2 or 3 carbon atoms, and 1.5 to 70 percent by weight of water. The amount of water in the aqueous-alcohol mixture must also be at least 1 percent of the total weight of the stick vehicle.

The polydiorganosiloxane-polyoxyalkylene copolymer contains at least one polydimethylsiloxane segment and at least one polyoxyalkylene segment, the polydiorganosiloxane segment consisting essentially of $R_nSiO_{(4-n)/2}$ units wherein n has a value of from 0 to 3, inclusive, there being an average value of approximately two R radicals per silicon for all siloxane units in the copolymer, and R denotes a radical selected from the group consisting of methyl, ethyl, vinyl, phenyl and a divalent radical bonding the polyoxyalkylene segment to the polydiorganosiloxane segment, at least 95 percent of all R radicals being methyl. The polyoxyalkylene segment has an average molecular weight of 1000 to about 5000 and consists

of from 0 to 60 mol percent polyoxypropylene units and from 40 to 100 mol percent polyoxyethylene units. A terminal portion of the polyoxyalkylene segment is bonded to the polydiorganosiloxane segment. The weight ratio of polydiorganosiloxane segments to polyoxyalkylene segments in the copolymer has a value of from 2 to 8.

The present invention is based on the discovery that a specific class of polydiorganosiloxane-polyoxyalkylene copolymers will act to compatibilize cyclic dimethylsiloxanes in aqueous-alcoholic solutions. It was further found that sodium stearate was soluble in such alcoholic-cyclic dimethylsiloxane mixtures and that appropriate amounts would gel the compositions and thus provide stick vehicles suitable for conveying skin care substances to skin.

A stick vehicle is prepared according to the present invention by first forming a solution or compatible mixture of the aqueous-alcohol and silicone components of the vehicle and then adding sodium stearate to gel the liquid mixture into a solid shaped article. Of course, any skin care substance that is to be applied to skin by use of the stick vehicle is added to the liquid mixture prior to gelling.

The exact form of the compatible mixture of aqueous-alcohol and silicone components is not known. The appearance of the mixtures may vary from clear and solution-like to translucent or even hazy and emulsion-like. For the purposes of this invention it is only necessary that the mixture be stable so that the components do not separate into distinct phases during the gelling process or subsequent use of the stick vehicle. Although the invention should not be limited by this theory, it is believed that the aqueous-alcoholic components are emulsified into the silicone components such that small, discontinuous domains of aqueous-alcoholic components are spread evenly throughout a continuous phase of silicone components.

The aqueous-alcoholic portion of the stick vehicle contains polyhydric alcohol, water, and optionally monohydric alcohol. The most useful alcohols have two to three carbon atoms and include ethanol, propanol, isopropanol, glycerol, and propylene glycol. It is anticipated that lower polyglycols having two to four glycol units could also be used in the alcoholic portion of the stick formulation.

The stick vehicle contains from 15 to 70 percent by weight of aqueous-alcohol mixture. The aqueous-alcohol mixture consists essentially of 20 to 70 percent by weight of polyhydric alcohol, preferably propylene glycol, 0 to 68.5 percent by weight of monohydric alcohol, preferably ethanol, and 1.5 to 70 percent by weight of water. The amount of water in the aqueous-alcohol mixture must also be at least 1 percent of the total weight of the stick vehicle. It has been found that sodium stearate will not gel the compositions without this small amount of water.

More preferred stick vehicles contain from 3 to 30 percent by weight, based on the total weight of the stick vehicle, of monohydric alcohol, and from 5 to 45 percent by weight, based on the total weight of the stick vehicle, of a polyhydric alcohol. These preferred stick vehicles contain low levels of water such as about 1 to 5 percent by weight of the stick vehicle. Stick vehicles with these low levels of water do not feel wet or sticky when applied to skin. Instead, these sticks are more typically described as providing a silky, smooth feeling that is very aesthetically pleasing. It is even more preferred, that the stick vehicle contain 10 to 25 percent by weight of monohydric alcohol and 15 to 30 percent by weight of polyhydric alcohol.

The required amounts of water may be introduced into the formulation along with other components, so that separate additions of water may not be required. For instance, 95% ethanol can be used in the vehicle and the water thereby introduced would be sufficient to meet the minimum requirements if 20 percent or more ethanol is used.

The silicone portion of the vehicle comprises cyclic polydimethylsiloxanes and a polydiorganosiloxane-polyoxyalkylene copolymer which compatibilizes the mixture of silicone and aqueous-alcoholic components. Cyclic polydimethylsiloxanes, often referred to as volatile silicones in the cosmetic art, have the formula $[-(CH_3)_2SiO]_x$ wherein x denotes 3, 4, 5, or 6. Volatile silicones are well known, commercially available products. The stick vehicles may contain from 10 to 70 percent by weight, based on the total weight of the vehicle, of cyclic polydimethylsiloxanes. Generally, it is preferred to employ from 35 to 60 percent by weight of cyclic polydimethylsiloxanes in the vehicles to provide the most improved aesthetics with minimum compatibility problems.

A polydiorganosiloxane-polyoxyalkylene copolymer is used in the vehicle to compatibilize the mixture of volatile silicone and alcohol components. The particular type of polydiorganosiloxane-polyoxyalkylene copolymers useful in this invention is a known material which has previously been used as a surfactant to stabilize emulsions of water in silicone oil. Such copolymers are described, for example, in U.S. Patent No. 4,265,878 which further describes the copolymers and methods of preparation.

Briefly, the polydiorganosiloxane-polyoxyalkylene copolymer is characterized as containing at least one polydiorganosiloxane segment and at least one polyoxyalkylene segment. The polydiorganosiloxane segment consists essentially of $R_n\ SiO_{(4-n)/2}$ units wherein n has a value of from 0 to 3, inclusive. There is an average of approximately two R radicals per siloxane unit in the copolymer with each R denoting a radical selected from the group consisting of methyl, ethyl, vinyl, phenyl and a divalent radical bonding the polyoxyalkylene segment to the polydiorganosiloxane segment.

To provide the compatibility needed in this invention, at least 95 percent of the R radicals must be methyl. In more preferred copolymers the polydiorganosiloxane segment consists essentially of dimethylsiloxane units and segment-terminating trimethylsiloxane units.

Useful polyoxyalkylene segments have an average molecular weight of 1000 to about 5000 and consist of from 0 to 60 mol percent polyoxypropylene units and from 40 to 100 mol percent polyoxyethylene units. Polyoxyalkylene segments consisting of 40 to 60 mol percent of polyoxypropylene units and 40 to 60 mol percent polyoxyethylene units are more preferred with segments consisting of an equimolar mixture of polyoxypropylene and polyoxyethylene units being most preferred. A terminal portion of each polyoxyalkylene segment is bonded to the polydiorganosiloxane segment.

In order to provide the proper compatibilizing effect, the proportion of polydiorganosiloxane and polyoxyalkylene segments of the copolymer must be within a certain ratio. Specifically, useful copolymers have a weight ratio of polydiorganosiloxane segments to polyoxyalkylene segments in the copolymer of from 2 to 8, or more preferably 2 to 4.

For example, a preferred copolymer, with a weight ratio of about 2.8 polydiorganosiloxane segments to polyoxyalkylene segments, is characterized by the average formula

$$\begin{array}{l}(CH_3)_3SiO[(CH_3)_2SiO]_{400}[CH_3\underset{|}{S}iO]_4Si(CH_3)_3\\ \qquad\qquad\qquad\qquad\qquad (CH_2)_3(CH_2CH_2O)_{25}(\underset{|}{C}HCH_2O)_{25}OOCCH_3.\\ \qquad\qquad\qquad\qquad\qquad\qquad\qquad\qquad\quad CH_3\end{array}$$

Typically from 0.5 to 10 percent by weight of the copolymer, based on the total weight of the vehicle, is employed in the invention, with 2 to 8 percent generally being preferred. It has also been found that for acceptable stability, the amount of copolymer in the vehicle should also be at least 5 percent of the weight of cyclic polydimethylsiloxanes present in the vehicle. In other words when low amounts such as 10 percent of cyclic polydimethylsiloxanes are present, 0.5 percent copolymer can be used, but when higher amounts such as 50 percent cyclic polydimethylsiloxanes are present, about 2.5 percent of copolymer is generally required. The optimum amount of copolymer in a given formulation will also vary depending on the amount of cyclic polydimethylsiloxanes present. Usually one part copolymer for each 8 to 15 parts of cyclic polydimethylsiloxanes is preferred.

The vehicles of this invention are gelled to a non-flowable state by addition of sodium salts of higher fatty acids. Sodium stearate is especially suitable as the gelling agent for the vehicles of this invention. The amount of sodium stearate used may vary from 3 to 10 percent by weight, based on the total weight of the vehicle. Generally, 4 to 8 percent of sodium stearate yields sticks having a degree of firmness particularly suitable for deodorant sticks.

The vehicles of the present invention can also contain small amounts of other components to modify or improve characteristics of the stick to satisfy preferences in specific use situations. For example, to reduce syneresis, polyoxyalkylene ethers of fatty alcohols may be included in amounts of 0.1 to about 8 percent by weight based on the total vehicle weight. Useful ethers include, but are not limited to, polyoxypropylene (3) myristyl ether, polyoxyethylene (2) cetyl ether, and polyoxypropylene (10) cetyl ether. Alternatively, small amounts, such as 0.1 to 8 percent by weight, of other nonionic organic surface active agents may be employed to further stabilize the vehicles of this invention. Examples of nonionic surface active agents include but are not limited to polyoxyethylene (20) sorbitan monolaurate, polyoxypropylene (20) myristal ether propionate, and polyoxyethylene (16) polyoxypropylene (12) monobutyl ether.

The stick vehicles of this invention can be used for topical delivery of a wide range of skin care substances. Because active skin care substances are typically delivered in a dilute form, and only small amounts need be dispersed in the stick vehicle, the particular type of skin care substance employed has little effect on the overall stick vehicle characteristics. Examples of skin care substances which may be delivered in the vehicles of this invention include, but are not limited to, skin-conditioning components, such

as humectants, moisturizers, and emollients; skin-protecting components, such as sunscreens, barrier oils and resins, and insect repellants; topical medicaments, such as steroids, vitamins, anesthetics, antibiotics, and penetrants; hygiene components, such as antiseptics and deodorants; and cosmetic components, such as colorants, fragrances, and bleaches.

Stick vehicles according to the present invention provide improvements in aesthetics or feel on the skin, are non-irritating, and do not leave a tacky residue on the skin after application. The sticks allow exceptionally smooth and even application of active substances since they glide very easily over the skin surface.

The following examples are presented to illustrate the invention to those skilled in the art and should not be construed as limiting the invention, which is properly delineated in the appended claims. All proportions by parts or percents are by weight unless otherwise stated.

Example 1

Three cosmetic stick vehicles were prepared by mixing propylene glycol, ethanol (95%), cyclic polydimethylsiloxane, polydimethylsiloxane-polyoxyalkylene copolymer (dimethicone copolyol), water, and sodium stearate in the proportions indicated in Table 1. The cyclic polydimethylsiloxane that was used in this and the following examples is a commercially available mixture of a major amount of octamethylcyclotetrasiloxane and minor amounts of larger cyclic dimethylsiloxanes. The polydimethylsiloxanepolyoxyalkylene copolymer that was used in this and the following examples generally conforms to the average formula

$$(CH_3)_3SiO[(CH_3)_2SiO]_{400}[CH_3SiO]_4Si(CH_3)_3$$

$$(CH_2)_3(CH_2CH_2O)_{25}(CHCH_2O)_{25}OOCCH_3.$$

$$CH_3$$

The cyclic polydimethylsiloxane, polydimethylsiloxane-polyoxyalkylene copolymer, and water were first combined to form an initial mixture. Propylene glycol and ethanol (95%) were then added and vigorously mixed with the silicone components. In each case, a clear, solution-like mixture resulted.

Each mixture was heated to about 70°C and the sodium stearate was stirred into it. The solution was poured into molds and allowed to cool. In each case a solid firm stick was obtained which was judged acceptable for cosmetic and skin care applications. The cosmetic sticks were generally translucent in appearance. Some syneresis was observed in formulations A and B having a higher ethanol content.

Example 2

Cosmetic stick vehicles were prepared as described in Example 1 with the proportion of components indicated in Table 2. In this case the mixture of propylene glycol and ethanol with the silicone components, was not completely clear. The mixtures tended to be somewhat translucent or hazy but were stable and did not separate upon standing.

The mixtures were solidified by adding sodium stearate as described in Example 1. In each case, a solid firm stick was obtained which was judged acceptable for cosmetic applications. The cosmetic sticks were generally opaque or hazy in appearance. Although within acceptable limits, somewhat more syneresis was observed in these sticks than the sticks of Example 1.

Example 3

Cosmetic stick vehicles were prepared as described in Example 1 with the proportion of components indicated in Table 3. In these proportions, the mixture of propylene glycol and ethanol with the silicone components, did not form a stable homogeneous composition. Soon after mixing was stopped, the initially cloudy, milky composition separated into distinct phases.

Sodium stearate was added at 70°C to the continuously agitated compositions. The compositions were cooled in molds to form solid sticks. These sticks prepared from unstable compositions exhibited more syneresis than sticks prepared in Examples 1 and 2 and were judged less acceptable for cosmetic vehicle purposes.

Example 4

This example provides cosmetic stick vehicles within the scope of the invention which contain small amounts of skin care active components or other components to modify or improve characteristics of the stick.

Cosmetic stick vehicles were prepared as described in Example 1 by mixing 22.5 parts propylene glycol, 22.5 parts ethanol (95%), 39.4 parts cyclic polydimethylsiloxane, 4.7 parts polydimethylsiloxane-polyoxyalkylene copolymer, 0.9 part water, 5 parts of either polyoxypropylene (10) cetyl ether or polyoxypropylene (3) myristyl ether, and 5 parts of sodium stearate. In either case a solid firm stick was obtained which was judged acceptable for cosmetic applications. The cosmetic sticks exhibited reduced syneresis and improved clarity but were still somewhat hazy in appearance.

A deodorant stick was prepared using the above formulation with polyoxypropylene (3) myristyl ether by adding 0.2 part of 5-chloro-2-(2,4-dichlorophenoxy)phenol to act as a bacteriostat.

Example 5

Additional cosmetic stick vehicles were prepared as described in Example 1 with the proportion of components indicated in Table 4. In each case a stick vehicle was obtained which was judged suitable for topical delivery of active skin care components. The most preferred sticks having the lowest levels of separation of components were obtained with formulations J, K, and M. Formulations G, H, L, and N exhibited slight liquid separation upon standing and formulation I was less preferred because it exhibited somewhat more liquid separation.

Example 6

This example provides cosmetic stick vehicles within the scope of the invention which contain small amounts of skin care active components or other components to modify or improve characteristics of the stick.

Cosmetic stick vehicles were prepared as described in Example 1. In addition to the components indicated in Table 5, each formulation also contained 1.5 percent of polyoxyethylene (2) cetyl ether and 3.6 percent of polyoxyethylene (20) sorbitan monolaurate as cosurfactants to further improve the stability and reduce syneresis of the stick vehicle. Formulations O, P, and S also contained 0.1 percent of 5-chloro-2-(2,4-dichlorophenoxy)phenol which acts as a bacteriostat when applied to the skin surface. Formulations O, P, and S are useful as deodorant sticks.

Formulations Q and R also contained 3 percent of octyl dimethyl paba (the ester of 2-ethylhexyl alcohol and p-aminobenzoic acid) which acts as a sunscreen when topically applied to the skin surface. Formulations Q and R are useful as sunscreen sticks.

TABLE 1

Weight Percent of Components in Cosmetic Stick Formulations

| Formulation | Propylene Glycol | Ethanol | Dimethicone Copolyol | Cyclic Polydimethyl-siloxane | Sodium Stearate | Water |
|---|---|---|---|---|---|---|
| A | 23.6 | 23.6 | 5 | 41.4 | 5.5 | 0.9 |
| B | 28 | 25.2 | 4.4 | 36.7 | 5.0 | 0.8 |
| C | 9.5 | 9.5 | 8.0 | 66.6 | 5.0 | 1.4 |

TABLE 2

Weight Percent of Components in Cosmetic Stick Formulations

| Formulation | Propylene Glycol | Ethanol | Dimethicone Copolyol | Cyclic Polydimethyl-siloxane | Sodium Stearate | Water |
|---|---|---|---|---|---|---|
| D | 23.8 | 4.8 | 7.0 | 58.2 | 5 | 1.3 |
| E | 18.9 | 14.7 | 6.5 | 53.8 | 5 | 1.2 |
| F | 40.4 | 14.3 | 4.2 | 35.4 | 5 | 0.8 |

TABLE 3

Weight Percent of Components in Cosmetic Stick Formulations

| Formulation | Propylene Glycol | Ethanol | Dimethicone Copolyol | Cyclic Polydimethyl-siloxane | Sodium Stearate | Water |
|---|---|---|---|---|---|---|
| G | 45.1 | 4.8 | 4.7 | 39.5 | 5 | 0.9 |
| H | 66.5 | 4.8 | 2.5 | 20.8 | 5 | 0.5 |

TABLE 4

Weight Percent of Components in Cosmetic Stick Formulations

| Formulation | Propylene Glycol | Ethanol | Dimethicone Copolyol | Cyclic Polydimethyl-siloxane | Sodium Stearate | Water |
|---|---|---|---|---|---|---|
| G | 20 | 20 | 3.7 | 30.7 | 5 | 20.6 |
| H | 20 | - | 3.7 | 30.7 | 5 | 40.6 |
| I | 25 | 25 | 3.7 | 30.7 | 5 | 10.6 |
| J | 27.5 | 27.5 | 3.7 | 30.7 | 5 | 5.6 |
| K | 40 | 0 | 3.7 | 30.7 | 5 | 20.6 |
| L | 40 | 10 | 3.7 | 30.7 | 5 | 10.6 |
| M | 40 | 20 | 3.7 | 30.7 | 5 | 0.6 |
| N | 20 | 40 | 3.7 | 30.7 | 5 | 0.6 |

TABLE 5

Weight Percent of Components in Cosmetic Stick Formulations

| Formulation | Propylene Glycol | Ethanol | Dimethicone Copolyol | Cyclic Polydimethyl-siloxane | Sodium Stearate | Water |
|---|---|---|---|---|---|---|
| O | 20.4 | 15.3 | 2.1 | 37.5 | 8 | 10.6 |
| P | 15.3 | 20.4 | 2.1 | 37.5 | 8 | 10.6 |
| Q | 18.1 | 15.6 | 2.1 | 37.5 | 8 | 10.4 |
| R | 20.4 | 13.8 | 2.1 | 37.5 | 7 | 10.6 |
| S | 20 | 15 | 2.1 | 37.5 | 8 | 12.2 |

## Claims

1. A stick vehicle comprising

(A) from 15 to 70 percent by weight of an aqueous-alcohol mixture, the aqueous-alcohol mixture consisting essentially of 20 to 70 percent by weight of a polyhydric alcohol having 2 or 3 carbon atoms, 0 to 68.5 percent by weight of a monohydric alcohol with 2 or 3 carbon atoms, and 1.5 to 70 percent by weight of water, with the proviso that the amount of water is at least 1 percent by weight of the total weight of the stick vehicle;

(B) from 10 to 70 percent by weight of cyclic polydimethylsiloxane;

(C) from 3 to 10 percent by weight of sodium stearate; and

(D) from 0.5 to 10 percent by weight of polydiorganosiloxane-polyoxyalkylene copolymer, with the proviso that the amount of copolymer is at least 5 percent of the weight of cyclic polydimethylsiloxane, the copolymer containing at least one polydimethylsiloxane segment and at least one polyoxyalkylene segment, the polydiorganosiloxane segment consisting essentially of $R_nSiO_{(4-n)/2}$ units wherein n has a value of from 0 to 3, inclusive, there being an average value of approximately two R radicals per silicon for all siloxane units in the copolymer, and R denotes a radical selected from the group consisting of methyl, ethyl, vinyl, phenyl and a divalent radical bonding said polyoxyalkylene segment to the polydiorganosiloxane segment, at least 95 percent of all R radicals being methyl; and the polyoxyalkylene segment having an average molecular weight of 1000 to about 5000 and consisting of from 0 to 60 mol percent polyoxypropylene units and from 40 to 100 mol percent polyoxyethylene units, at least one terminal portion of the polyoxyalkylene segment being bonded to the polydiorganosiloxane segment, the weight ratio of polydiorganosiloxane segments to polyoxyalkylene segments in the copolymer having a value of from 2 to 8.

2. The stick vehicle according to claim 1 wherein the polyhydric alcohol is propylene glycol and the monohydric alcohol is ethanol.

3. The stick vehicle according to claim 2 wherein the polydiorganosiloxane segment of the copolymer consists essentially of dimethylsiloxane units and segment-terminating trimethylsiloxane units and the polyoxyalkylene segments consist of 40 to 60 mol percent polyoxypropylene units and 40 to 60 mol percent polyoxyethylene units.

4. The stick vehicle according to claim 3 wherein the cyclic polydimethylsiloxane is selected from the group consisting of octamethylcyclotetrasiloxane and decamethylcyclopentasiloxane.

5. The stick vehicle according to claim 2 wherein the vehicle further comprises 0.1 to 8 percent by weight of a polypropylene glycol ether of a fatty alcohol.

6. The stick vehicle according to claim 1 wherein a skin care substance is distributed throughout the stick.

7. The stick vehicle according to claim 6 wherein the skin care substance is a bacteriostat.

8. The stick vehicle according to claim 7 wherein the bacteriostat is 5-chloro-2-(2,4-dichlorophenoxy)-phenol.